# FASCICULE DE BREVET EUROPEEN

(11) **EP 0 575 605 B1**
(45) Date de publication et mention de la délivrance du brevet: **27.11.1996**
(21) Numéro de dépôt: 93904077.0
(22) Date de dépôt: 13.01.1993
(51) Int. Cl.: A61K 7/00, A61K 7/13, C09B 69/10

(54) **PRODUIT A BASE DE PARTICULES MINERALES OU ORGANIQUES PORTANT UN PRODUIT INDOLINIQUE**
MITTEL AUF BASIS VON ANORGANISCHEN ODER ORGANISCHEN PARTIKELN MIT EINER INDOLINVERBINDUNG
MINERAL OR ORGANIC PARTICLE BASED PRODUCT COMPRISING AN INDOLINE PRODUCT

(30) Priorité: 16.01.1992 FR 9200417
(43) Date de publication de la demande: 29.12.1993
(73) Titulaire: L'OREAL, 75008 Paris (FR)
(72) Inventeur: LAGRANGE, Alain, F-78400 Chatou (FR); ANDREAN, Hervé, F-75014 Paris (FR); JUNINO, Alex, F-93190 Livry-Gargan (FR)
(74) Mandataire: Casalonga, Axel
(86) Numéro de dépôt international: FR9300031
(87) Numéro de publication internationale: WO9313745

(56) Documents cités:
- EP-A- 0 379 409
- EP-A- 0 462 857
- GB-A- 2 207 153

## Description

La présente invention est relative à un produit sous forme de particules minérales ou organiques, comportant dans ou sur les particules, un produit indolinique, son procédé de préparation et son utilisation en cosmétique, notamment pour le maquillage des phanères et/ou de la peau et la protection de l'épiderme humain contre les rayonnements UV.

On utilise généralement dans les compositions de maquillage pour la peau et les phanères, des pigments à base de composés métalliques tels que par exemple des oxydes de fer noir et brun. Ces pigments ne sont cependant pas totalement inoffensifs et on recherche de ce fait des pigments susceptibles de présenter moins de problèmes dans leur application cosmétique.

On souhaite également à certaines occasions pouvoir conférer aux cheveux une coloration qui puisse être éventuellement enlevée très rapidement.

La demanderesse vient de découvrir qu'il était possible de préparer in vitro un produit sous forme d'une poudre formée de particules minérales ou organiques, comportant dans et/ou sur les particules un ou plusieurs produits indoliniques résultant de la polymérisation oxydative d'au moins un composé de la famille des indolines définie ci-après.

Elle a découvert que l'utilisation de ce produit sous forme de particules était particulièrement avantageuse dans la mesure où ces particules une fois introduites dans un milieu cosmétiquement acceptable, se répartissaient bien dans la composition qui s'étale facilement sur les phanères ou la peau et présente un pouvoir couvrant important.

Elle a également constaté que le produit ainsi obtenu présentait un coefficient d'absorption des radiations ultraviolettes particulièrement intéressant.

Enfin, la présence du produit indolinique résultant de la polymérisation oxydative d'au moins un composé indolinique, permet d'obtenir des colorations du produit final particulièrement intéressantes dans leur utilisation en cosmétique, dans la mesure où les colorations pouvant être obtenues avec les pigments disponibles et compatibles avec l'application cosmétique, étaient relativement limitées. Les colorations ainsi obtenues sont particulièrement stables à la lumière.

On appellera par la suite "produit indolinique" ou "polymère indolinique", le produit obtenu par oxydation d'au moins une indoline définie ci-après.

La présente invention a donc pour objet une poudre constituée de particules minérales ou organiques, comportant dans et/ou sur les particules, un produit indolinique.

Un autre objet de l'invention est constitué par la préparation d'une telle poudre.

L'invention a également pour objet l'application cosmétique de telles poudres, notamment dans les produits de maquillage de la peau, des phanères et de la protection de l'épiderme humain contre les rayonnements UV.

On appelle "phanères", conformément à l'invention, les cheveux, les poils tels que cils, sourcils et les ongles.

D'autres objets de l'invention apparaîtront à la lecture de la description et des exemples qui suivent.

Le produit conforme à l'invention est essentiellement caractérisé par le fait qu'il se présente sous forme d'une poudre constituée de particules minérales ou organiques, dont la dimension la plus grande est inférieure à 200 micrometres et comportant dans et/ou sur les particules, un produit indolinique synthétique formé in situ par polymérisation oxydative d'au moins une indoline.

Le produit indolinique résulte de l'oxydation d'au moins un composé de la famille des indolines, répondant à la formule : 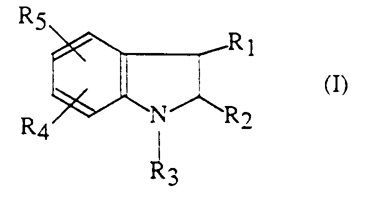 dans laquelle :
R₁ et R₃ représentent, indépendamment l'un de l'autre, un atome d'hydrogène ou un groupement alkyle en C₁-C₄;
R₂ représente un atome d'hydrogène, un groupement alkyle en C₁-C₄, un groupement carboxyle ou alcoxy(C₁-C₄)carbonyle;
R₄ désigne un atome d'hydrogène, un groupement alkyle en C₁-C₄, hydroxyle, alcoxy(C₁-C₄), amino, alkylamino en C₁-C₁₀ ou halogène;
R₅ désigne un atome d'hydrogène, un groupement hydroxyle, alcoxy en C₁-C₄ ou amino;
au moins un des radicaux R₄ ou R₅ désignant un groupement hydroxyle, alcoxy ou amino; sous réserve que lorsque R₅ désigne un groupement amino, R₄ ne peut désigner un radical alkylamino;
R₄ et R₅ peuvent également former un cycle alkylènedioxy en C₁-C₂ et sont en position 5 et 6;
ainsi que les sels correspondants.

Parmi les composés répondant à la formule (I), les composés préférés utilisés conformément à l'invention, sont choisis parmi la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline et la 5,6-dihydroxy 2-carboxyindoline.

Les sels des composés précités sont des sels cosmétiquement acceptables choisis en particulier parmi les chlorhydrates, bromhydrates, sulfates, méthanesulfonates. Les bromhydrates des composés ci-dessus sont particulièrement préférés.

Les produits indoliniques peuvent aussi résulter de la cooxydation d'au moins une indoline définie et d'au moins un dérivé d'indole. Ce dernier peut être choisi parmi les mono- ou dihydroxyindoles ou les aminoindoles, tels que décrits plus particulièrement dans le brevet EP-A-239 826 et les demandes de brevet EP-A-425 345 et GB-A-2 224 754.

Ces indoles répondent plus particulièrement à la formule : 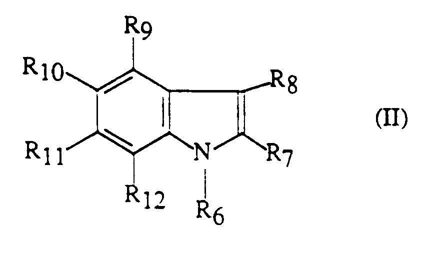 dans laquelle :
R₆ et R₈ désignent, indépendamment l'un de l'autre, un atome d'hydrogène ou un groupe alkyle en C₁-C₄;
R₇ représente un atome d'hydrogène, un groupement alkyle en C₁-C₄, un groupement carboxyle ou un groupement alcoxy(C₁-C₄) carbonyle;
R₉ et R₁₂ désignent, indépendamment l'un de l'autre, un atome d'hydrogène, un groupement hydroxy, un groupe alkyle en C₁-C₄, amino, alcoxy(C₁-C₄), acyl(C₂-C₄)oxy, acyl(C₂-C₄)amino;
R₁₀ désigne hydrogène, un groupement hydroxy, alcoxy(C₁-C₄), alkyle(C₁-C₄), halogène, amino, acyl(C₂-C₁₄)oxy, acyl(C₂-C₄)amino, triméthylsilyloxy;
R₁₁ désigne hydrogène, un groupement hydroxy, alcoxy(C₁-C₄), amino, acyl(C₂-C₄)oxy, acyl(C₂-C₄)amino, triméthylsilyloxy, hydroxyalkyl(C₂-C₄)amino;
R₁₀ et R₁₁ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy;
au moins l'un des groupements R₉ à R₁₂ représente un groupement OZ ou NHR, un seul au plus des groupements R₉ à R₁₂ désignant NHR;
et au plus deux des groupements R₉ à R₁₂ désignent OZ, dans le cas où Z désigne hydrogène, ces groupements sont en position 5 et 6;
et au moins un des groupements R₉ à R₁₂ représente hydrogène, dans le cas où un seul de ces groupements désigne hydrogène, un seul groupement parmi R₉ à R₁₂ désigne alors NHR ou OZ, et les autres groupements désignent alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants.

Les indoles sont choisis parmi le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, 5-aminoindole, 6-aminoindole, 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole.

Lors de la cooxydation, on peut utiliser jusqu'à 50% en mole de dérivés indoliques par rapport au nombre total de moles de dérivés à oxyder.

Les particules utilisées conformément à l'invention sont des particules minérales sous forme lamellaire ou non lamellaire, organiques lamellaires ou non lamellaires colorées ou non. Ces particules ont une granulométrie moyenne comprise entre 0,01 et 200 micromètres.

Les particules minérales non lamellaires utilisables conformément à l'invention, sont des particules minérales inertes ayant une granulométrie inférieure à 20 micromètres et de préférence inférieure à 10 micromètres et plus particulièrement inférieure ou avoisinant 5 micromètres

De telles particules sont en particulier choisies parmi les particules de carbonate de calcium, de silice ou d'oxyde de titane ayant la granulométrie définie ci-dessus.

Les particules lamellaires utilisées conformément à l'invention, sont des particules minérales ou organiques se présentant sous forme de feuillets éventuellement stratifiés.

Ces feuillets se caractérisent par une épaisseur plus faible que la plus grande dimension. De préférence, le rapport entre la plus grande dimension et l'épaisseur est compris entre 2 et 100. La dimension la plus grande est généralement inférieure à 50 micromètres.

Les particules de structure lamellaire sont choisies en particulier parmi les produits suivants : la L-lauroyllysine telle que le produit vendu sous la dénomination AMIHOPE L.L* par la Société AJINOMOTO*; les microparticules de céramique éventuellement recouvertes de poudre de zirconium telles que les produits vendus sous les appellations TORAYCERAM* ZP 550 et ZP 4000 par la Société TORAY*; le dioxyde de titane lamellaire tel que les produits vendus sous les dénominations LUXELEN* SILK D et LUXELEN* SS par la Société SUMITOMO*, le talc lamellaire, le nitrure de bore tel que les produits vendus sous les dénominations Nitrure de bore SF ou SHP par les Sociétés WACKER* et KAWASAKI*; le mica lamellaire tel que le produit vendu sous la dénomination MICA* CONCORD* 1000 par la Société SCIAMA*; l'oxychlorure de bismuth tel que le produit vendu sous la dénomination PEARL GLO* par la Société MALLINCKRODT*; l'oxyde de fer transparent rouge tel que le produit vendu sous la dénomination CAPPOXYT* 4435 B par la Société CAPPELLE*.

La dimension des particules de structure lamellaire utilisées conformément à l'invention, est de préférence inférieure à 50 micromètres et en particulier inférieure à 25 micromètres. Leur dimension est généralement supérieure à 0,5 micromètres. Elle est comprise en particulier entre 1 et 20 micromètres. Ces particules ont une épaisseur généralement supérieure à 0,01 micromètres. Comme indiqué plus haut, ces particules lamellaires peuvent se présenter sous forme d'une structure stratifiée.

On peut également utiliser selon l'invention, des particules colorées. Ce sont des particules minérales non blanches, colorées, constituées de sels métalliques, insolubles dans le milieu cosmétique, utilisables en cosmétique, référencées dans le Color Index sous le chapitre "Inorganic Colouring Matters" et portant les numéros 77000 à 77947, à l'exclusion des pigments blancs. Ces particules minérales colorées peuvent être constituées d'un seul pigment ou d'un mélange de pigments. Elles peuvent aussi se présenter sous forme de pigments nacrés ou interférentiels.

Les particules minérales colorées sont choisies de préférence parmi les oxydes de fer, le bleu outremer (qui est un sulfosilicate complexe), les oxydes de chrome, le violet de manganèse (qui est un pyrophosphate d'ammonium et de manganèse) et le bleu de Prusse (qui est un ferricyanure de fer).

La granulométrie des particules de la poudre colorée en utilisant des particules minérales colorées comportant le produit indolinique, dépend de la granulométrie des particules colorées utilisées et peut varier dans de larges limites allant de 0,01 à 150 micrometres.

Ainsi, lorsque la particule minérale colorée de départ est un pigment nacré ou interférentiel, la granulométrie des particules de la poudre conformes à l'invention, varie entre 10 et 150 micrometres.

Par contre, lorsque la particule minérale colorée est un sel métallique (oxydes de fer, de chrome, sels de fer, de manganèse), la granulométrie des particules de la poudre conformes à l'invention, est généralement comprise entre 0,01 et 5 micromètres.

Les particules organiques non lamellaires utilisées conformément à l'invention, sont des particules fines de polymères. Le produit obtenu avec ces particules organiques conforme à l'invention, est caractérisé par le fait qu'il est constitué de particules de polymères ayant une granulométrie inférieure à 100 micrometres et comportant, en surface et/ou dans le réseau polymère, un produit indolinique résultant de la polymérisation oxydative d'au moins un composé de la famille des indolines répondant à la formule (I) définie précédemment et éventuellement d'une cooxydation avec un composé indolique.

La granulométrie de ces particules organiques est généralement supérieure à 0,01 micromètre et de préférence comprise entre 0,01 et 50 microns et en particulier entre 0,1 et 20 micromètres. Elles sont de préférence sphérique.

Les polymères utilisables selon l'invention sont des polymères essentiellement insolubles dans le milieu réactionnel et sont choisis parmi les polymères naturels ou synthétiques, organiques ou inorganiques, à réseau réticulé cristallin ou amorphe, ayant un poids moléculaire compris entre 5000 et 5 000 000.

Le caractère essentiellement insoluble du polymère est justifié par des raisons essentiellement économiques dans la mesure où le produit indolinique doit se fixer sur un support particulaire solide, pour former le produit conforme à l'invention.

La solubilité des polymères dans le milieu réactionnel ne doit pas dépasser de préférence 10%.

Les polymères organiques ou synthétiques sont en particulier choisis parmi les polymères dérivés de la kératine, de la chitine ou de la cellulose et des polyamides ou des homo ou copolymères résultant de la polymérisation de monomères mono ou polyéthyléniques, aliphatiques ou aromatiques, à réseau réticulé, cristallin ou amorphe.

Les polymères dérivés de la kératine sont en particulier choisis parmi les kératines animales ou humaines, issues, par exemple, de matériaux choisis parmi les cheveux, la laine, la peau, les poils, les soies, les plumes, les écailles et plus particulièrement les sabots, la come, ou encoer la fibroïne de soie.

Ces matériaux sont de préférence lavés et/ou dégraissés, puis réduits en particules.

D'autres polymères dérivés de la kératine sont des kératines modifiées chimiquement, ayant un poids moléculaire compris entre 10 000 et 250 000, et en particulier la kératine partiellement hydrolysée (ou hydrolysat de kératine), obtenue à partir de peaux qui sont riches en produits soufrés et ayant un poids moléculaire compris entre 50 000 et 200 000. Cet hydrolysat est de préférence obtenu par hydrolyse alcaline modérée.

Des produits de ce type sont par exemple vendus sous la dénomination de KERASOL* par la Société CRODA*.

D'autres kératines modifiées sont les kératines sulfoniques d'un poids moléculaire compris entre 10 000 et 100 000, obtenues à partir de plumes d'oie ou de poulet ou plus avantageusement encore de sabots ou de come.

Cette kératine est obtenue par oxydation de tout ou partie des liaisons disulfures des groupements cystine de la kératine en groupements acide cystéique : SO₃H, l'oxydation étant avantageusement effectuée en milieu acide, tel que l'acide formique, au moyen d'un agent oxydant, tel que l'eau oxygénée.

Les polymères dérivés de la chitine sont constitués par la chitine qui est un polymère naturel, dont la source la plus importante se trouve dans la carapace des crustacés, tels que crabes, homards, langoustes, etc... La chitine est préparée selon un procédé décrit, notamment, dans l'ouvrage dez R.A.A. MUZZARELLI "CHITIN", édité chez PERGAMON PRESS OXFORD, 1977, pages 89-100 et 207-217. On peut également utiliser son dérivé désacétylé connu sous la dénomination de chitosane, obtenu par hydrolyse des groupements acétyle de la chitine.

Le chitosane, tel que proposé dans le commerce, est partiellement acétylé et contient 70 à 90% en poids de chitosane. On peut également l'utiliser sous forme de ses sels insolubles, tels que les sulfates et phosphates. Des produits de ce type sont vendus par exemple sous la dénomination de KYTEX* par la Société HERCULES*.

Les polymères cellulosiques sont choisis plus particulièrement parmi les celluloses microcristallines, tels que les produits vendus sous la dénomination AVICEL* par la Société FMC*.

Parmi les polymères synthétiques, on peut tout particulièrement citer le polyéthylène, le polypropylène, le polystyrène, le polyméthacrylate de méthyle, le polyméthacrylate de méthyle réticulé, tel que le produit vendu sous la dénomination MICROPEARL* M 305 par la Société SEPPIC*. D'autres polymères sont en particulier choisis parmi la poly-β-alanine réticulée, telle que décrite dans le brevet français 2 530 250, ou encore présentée avantageusement sous forme de microsphères présentant une très faible dispersité de taille, 85% en poids ayant une granulométrie comprise entre 28 et 46 micrometres. Ces poly-β-alanines sont obtenues suivant un procédé consistant à polymériser entre 60 et 100°C, et de préférence vers 80°C, de l'acrylamide dans un mélange de solvants t-butanol/toluène, dans des rapports compris entre 1:24 et 10:1 et de préférence entre 1:6 et 6:1, en présence d'un initiateur de polymérisation, d'un copolymère octadécène-anhydride maléique comme agent de suspension, puis à soumettre la suspension de poly-β-alanine obtenue à une réticulation à l'aide d'un dialdéhyde, tel que le glutaraldéhyde.

L'initiateur de polymérisation est de préférence du tertiobutylate de sodium ou de potassium (0,1 à environ 2 moles % par rapport à l'acrylamide).

Le glutaraldéhyde est utilisé sous forme d'une solution aqueuse entre 20 et 25% et dans une proportion comprise entre 1 et 15% en poids par rapport au poids d'acrylamide de départ.

On peut également utiliser à titre de polymères des produits connus sous la dénomination de microéponges, tels que des polymères réticulés de styrène/divinylbenzène ou de méthacrylate de méthyle/ diméthacrylate d'éthylèneglycol ou de stéarate de vinyle/divinylbenzène, tels que décrits dans les brevets WO-88/01164 et US-A-4690 825.

De tels polymères sont essentiellement constitués par des billes de polymères réticulés comportant un réseau interne de pores, capable de retenir le produit indolinique.

D'autres polymères de ce type sont des microsphères creuses d'un copolymère de chlorure de vinylidène et d'acrylonitrile, vendues sous la dénomination EXPANCEL* par la Société KEMA* NORD; des microsphères poreuses de polyamide 12, de polyamide 6 ou de copolyamide 6/12, vendues sous la dénomination ORGASOL* par la Société ATO-CHIMIE*. Ces microsphères ont de préférence une granulométrie comprise entre 10 et 50 micrometres.

On peut aussi utiliser des poudres de silicones qui sont des gommes, des résines et plus particulièrement des élastomères d'organosiloxanes.

Les produits conformes à l'invention peuvent être préparés suivant un procédé consistant essentiellement à mélanger à l'air et à une température de préférence ambiante et pouvant aller jusqu'à 100°C, au moins un composé indolinique de formule (I) et les particules minérales ou organiques décrites ci-dessus, dans un milieu essentiellement non-solvant des particules.

L'oxydation du composé indolinique de formule (I) peut s'effectuer en milieu aqueux ou eau-solvant, à l'air, à l'air en présence d'un agent alcalin et/ou d'un catalyseur métallique d'oxydation tel que l'ion cuivrique.

Si on n'utilise pas d'autre agent oxydant que l'oxygène de l'air, on opère à un pH de préférence alcalin, auquel cas, le pigment se forme progressivement et se fixe sur la surface des particules et/ou dans le réseau ou les pores de celles-ci.

Ces produits peuvent également être préparés en procédant à une formation immédiate du produit indolinique en utilisant un agent oxydant tel que le peroxyde d'hydrogène, les peracides et les persels.

On utilise de préférence l'acide periodique et ses sels hydrosolubles et dérivés, les peracides organiques et leurs sels, les permanganates et bichromates, tels que de sodium ou de potassium, l'hypochlorite de sodium, le ferricyanure de potassium, le persulfate d'ammonium, les chlorites alcalins, l'oxyde d'argent, le chlorure ferrique, l'oxyde de plomb, le nitrite de sodium, les sels de terres rares tels que de cérium.

On peut également utiliser des oxydants organiques choisis parmi les ortho et parabenzoquinones, les ortho et parabenzoquinones monoimines et diimines, les 1,2- et 1,4-naphtoquinones, les 1,2- et 1,4-naphtoquinones mono- ou diimines. L'oxydation peut également s'effectuer par addition d'iodure et de peroxyde d'hydrogène, l'iodure étant de préférence un iodure de métal alcalin, alcalino-terreux ou d'ammonium.

Le sel d'acide periodique préféré est le periodate de sodium.

Ces agents oxydants peuvent éventuellement être activés par un agent modificateur de pH.

Pour les produits destinés à une application cosmétique, on utilise de préférence comme agents oxydants le peroxyde d'hydrogène, l'acide periodique et ses sels, le permanganate de potassium, l'hypochlorite de sodium, le persulfate d'ammonium, le nitrite de sodium et le système iodure/peroxyde d'hydrogène.

L'ordre d'addition des composés intervenant dans la préparation du produit sous forme de particules, conforme à l'invention, a peu d'importance à la condition que l'on incorpore en dernier lieu l'agent oxydant quand celui-ci est utilisé sans agent modificateur de pH et dans le cas du système oxydant iodure/peroxyde d'hydrogène, on introduit en dernier lieu, soit le peroxyde d'hydrogène, soit l'iodure.

On peut également procéder à une oxydation par voie enzymatique en procédant à l'oxydation à l'aide d'une enzyme à activité oxydante ou peroxydante telle que la peroxydase de Raifort, la chloroperoxydase, la peroxydase du lait, la cytochrome C-peroxydase ou des enzymes peroxydantes, notamment l'hémoglobine, la methémoglobine, la myoglobine, la metmyoglobine. Cette oxydation enzymatique peut également s'effectuer en présence de tyrosinase avec l'oxygène de l'air.

Dans le cas où l'on utilise un agent modificateur de pH pour activer l'agent oxydant, on préfère ajouter en dernier lieu, soit l'agent oxydant, soit l'agent modificateur de pH.

Lorsqu'on utilise comme particule organique un hydrolysat de kératine, le pH du milieu peut de préférence être inférieur à 5 afin d'éviter la solubilisation de la kératine modifiée.

Lorsqu'on utilise une kératine sulfonique, le milieu est soit essentiellement alcoolique, soit aqueux, auquel cas, le pH doit être inférieur à 7.

Lorsqu'on utilise le chitosane, le milieu aqueux doit avoir de préférence un pH supérieur à 5.8.

Le milieu réactionnel utilisé doit être un milieu essentiellement non solvant de la particule organique ou minérale considérée. Il est de préférence constitué par de l 'eau et peut éventuellement être constitué par un mélange d'eau et d'un ou plusieurs solvants, tel que des alcools inférieurs en C₁-C₄, comme l'alcool éthylique, l'alcool propylique ou isopropylique, l'alcool tertiobutylique, les alkylèneglycols tels que l'éthylèneglycol, le propylèneglycol, les alkyléthers d'alkylèneglycols tels que les éthers monométhylique, monoéthylique et monobutylique de l'éthylèneglycol, les monométhyléthers du propylèneglycol et du dipropylèneglycol et le lactate de méthyle.

Le milieu doit par ailleurs pouvoir solubiliser le composé indolinique et éventuellement le ou les dérivés indoliques.

Lorsque le milieu est constitué par un mélange eau/solvant(s), le(s) solvant(s) est (sont) présent(s) dans des concentrations de préférence comprises entre 0,5 et 95% en poids par rapport au poids total de la composition et en particulier entre 2 et 50% en poids et de préférence entre 2 et 20% en poids.

Leur nature est choisie et leur proportion est ajustée en fonction des critères de solubilité des dérivés de la famille des indolines (I) définies ci-dessus, éventuellement des indoles de formule (II) en cas de cooxydation et du critère d'insolubilité des particules minérales ou organiques employées.

Dans le procédé conforme à l'invention, on utilise de préférence les composés de la famille des indolines dans des proportions pondérales comprises entre 0,1 et 10% et de préférence entre 0,5 et 7% en poids, la charge minérale ou organique représentant 0,075 à 70% en poids et de préférence 4 à 50% en poids par rapport au poids du milieu réactionnel, le reste du mélange étant généralement constitué par l'eau ou un mélange eau/solvant.

Les oxydants sont mis en oeuvre dans des quantités suffisantes pour oxyder le composé de la famille des indolines de formule (I) et éventuellement cooxyder le composé indolinique de formule (I) et le composé indolique de formule (II) pour former le produit indolinique sur ou dans les particules minérales ou organiques.

Lorsqu'on utilise l'ion iodure pour former le produit indolinique, celui-ci est utilisé de préférence dans des proportions de 0,07 à 4% et en particulier entre 0,7 et 3% en observant un rapport composé indolinique et éventuellement dérivé d'indole par rapport aux ions iodures compris entre 0,6 et 6.

Les proportions sont déterminées par rapport au poids du milieu réactionnel. Lorsqu'on veut réaliser une cooxydation d'un ou plusieurs dérivés indoliniques de formule (I) avec un ou plusieurs dérivés d'indole de formule (II), on procède de la même façon, en mélangeant simplement avant oxydation des dérivés à oxyder.

Le produit à base de particules comportant dans et/ou sur la particule le produit indolinique, conforme à l'invention, est utilisé en cosmétique, plus particulièrement dans des compositions de maquillage de la peau et/ou des phanères et de protection de l'épiderme humain.

La poudre sous forme de particules minérales ou organiques et comportant le produit indolinique tel que défini ci-dessus, peut être additionnée dans des supports cosmétiques classiques à une concentration comprise entre 0,05 et 35% en poids et de préférence entre 0,5 et 20% en poids par rapport au poids total de la composition pour conduire à des compositions cosmétiques protectrices de l'épiderme humain, des produits de maquillage tels que de maquillage des cils, des sourcils, de la peau, des cheveux ou des ongles, comme des fards à paupières, fards à joues, ligneurs encore appelés "eye-liners", des mascaras pour les cils et les sourcils, des vernis à ongles, ou encore des compositions de coloration temporaire des cheveux. Ces supports cosmétiques sont connus en eux-mêmes.

Le milieu utilisé dans ces différentes compositions cosmétiques est un milieu essentiellement non solvant des particules minérales ou organiques, comportant le produit indolinique.

On appelle milieu essentiellement non solvant, un milieu qui dissout moins de 1% en poids des particules minérales ou organiques comportant le produit indolinique.

Les compositions peuvent se présenter en particulier sous forme de lotion, de lotion épaissie, de gel, de crème, de lait, de poudre, de stick et éventuellement être conditionnées en aérosol et se présenter sous forme de mousse ou de spray.

Lorsque les compositions sont utilisées pour le maquillage de la peau, des cheveux, des cils et des sourcils, elles peuvent notamment se présenter sous forme solide ou pâteuse, anhydre ou aqueuse, comme des émulsions huile-dans-eau ou eau-dans-huile ou encore des suspensions. Ces compositions présentent l'avantage d'être stables et de présenter une bonne innocuité.

Lorsque les compositions sont utilisées pour la protection de l'épiderme humain contre les rayonnements UV, elles constituent des compositions dites "solaires" et elles peuvent se présenter sous forme de suspensions ou de dispersions dans des solvants ou des corps gras, ou encore sous forme d'émulsions telles que crèmes et laits, de pommades, de gels, de bâtonnets solides ou de mousses aérosols.

Dans tous les cas, lorsqu'elles sont utilisées sous forme d'émulsions, elles peuvent contenir en outre des agents tensio-actifs bien connus dans l'état de la technique, tels que des agents tensio-actifs anioniques, non-ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non-ioniques, amphotères, ou leurs mélanges, des propulseurs, des agents alcalinisants ou acidifiants.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies parmi les cires animales, fossiles, végétales, minérales ou de synthèse. On peut citer notamment les cires d'abeilles, les cires de Carnauba, de Candellila, de canne à sucre, du Japon, les ozokérites, la cire de Montan, les cires microcristallines, les paraffines.

Lorsque les compositions sont utilisées pour la coloration des ongles, elles se présentent sous forme de produits dits "vernis à ongles" contenant la poudre conforme à l'invention sous forme dispersée dans un solvant cosmétiquement acceptable contenant une ou plusieurs résines et des ingrédients habituellement utilisés dans ce type de produit.

Les compositions conformes à l'invention peuvent également contenir en plus des particules minérales ou organiques comportant le produit indolinique, tel que défini ci-dessus, d'autres pigments généralement utilisés en cosmétique, notamment des pigments colorés ou blancs, nacrés et/ou nacrants permettant de varier les colorations susceptibles d'être obtenues, ou d'augmenter la protection vis-à-vis des rayonnements ultraviolets. Dans ce dernier cas, on utilise de préférence des nanopigments d'oxydes métalliques tels que les oxydes de titane, de zinc, de cérium ou de zirconium, de diamètre moyen inférieur à 100 nm et de préférence compris entre 5 et 50 nm. Les pigments peuvent être enrobés ou non enrobés.

Les pigments enrobés sont des pigments qui ont subi un ou plusieurs traitements de surface de nature chimique, électronique, mécanochimique, et/ou mécanique avec des composés tels que décrits par exemple dans COSMETICS & TOILETRIES, Février 1990, Vol. 105, pages 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

L'invention a également pour objet un procédé de coloration temporaire des cheveux, de maquillage de la peau, des cils et sourcils ou des ongles, de la protection de l'épiderme humain contre les effets néfastes des rayonnements UV, mettant en oeuvre une poudre à base de particules minérales ou organiques comportant des produits indoliniques tels que définis ci-dessus, cette poudre étant appliquée directement ou au moyen de compositions cosmétiques telles que définies ci-dessus.

Les exemples suivants sont destinés à illustrer l'invention.

### EXEMPLES DE PREPARATION

### EXEMPLE 1

On solubilise 7,3 g (0,031 mole) de bromhydrate de 5,6-dihydroxyindoline dans 100 ml de solution aqueuse à 0,1% d'ammoniaque. On ajoute à ce mélange 45 g de poudre de polyamide 12 vendu sous la dénomination ORGASOL* 2002 D Natural COS par la Société ATOCHEM*. On agite pendant 15 minutes cette suspension, puis on la porte à 80°C. On ajoute alors 10,5 ml de soude 3N et on additionne en 30 minutes 28,6 g d'eau oxygénée contenant 2,3 g (0,067 mole) de peroxyde d'hydrogène en maintenant la température entre 80 et 85°C. L'addition terminée, on maintient la température à 80°C pendant 2 heures puis on refroidit le milieu réactionnel. On centrifuge le produit et on le lave à l'eau. Après lyophilisation, on obtient 42 g de poudre gris brun.

### EXEMPLE 2

On solubilise 7,3 g (0,031 mole) de bromhydrate de 5,6-dihydroxyindoline dans 100 ml de solution aqueuse à 0,1% d'ammoniaque. On ajoute à ce mélange 45 g de nitrure de bore vendu sous la dénomination SHP2 par la Société KAWASAKI*. On agite 15 minutes cette suspension puis on la porte à 80°C. On ajoute alors 10,5 ml de soude 3N et on additionne en 30 minutes 28,6 g d'eau oxygénée contenant 2,3 g (0,067 mole) de peroxyde d'hydrogène en maintenant la température entre 80 et 85°C. On procède ensuite comme à l'exemple 1. On obtient après lyophilisation, 41 g de poudre gris bleuté.

### EXEMPLE 3

On solubilise 7,3 g (0,031 mole) de bromhydrate de 5,6-dihydroxyindoline dans 100 ml de solution aqueuse à 0,1% d'ammoniaque. On ajoute à ce mélange 45 g d'oxyde de fer rouge non lamellaire qui est un mélange d'oxyde de fer jaune (CI 77492) et d'oxyde de fer brun (CI 77491). On procède ensuite comme à l'exemple 1. Après lyophilisation, on obtient 41 g de poudre brune.

### EXEMPLE 4

On solubilise 7,3 g (0,031 mole) de bromhydrate de 5,6-dihydroxyindoline dans 100 ml de solution aqueuse à 0,1% d'ammoniaque. On ajoute à ce mélange 45 g de bleu outremer (CI 77007). On procède ensuite comme à l'exemple 1. Après lyophilisation, on obtient 49 g de poudre bleu foncé.

### EXEMPLE 5

On solubilise 7,3 g (0,031 mole) de bromhydrate de 5,6-dihydroxyindoline dans 100 ml de solution aqueuse à 0,1% d'ammoniaque. On ajoute à ce mélange 45 g d'oxyde de chrome hydraté (CI 77289). On procède ensuite comme à l'exemple 1. Après lyophilisation, on obtient 41,5 g de poudre vert bleuté.

### EXEMPLE 6

On solubilise 7,3 g (0,031 mole) de bromhydrate de 5,6-dihydroxyindoline dans 100 ml de solution aqueuse à 0,1% d'ammoniaque. On ajoute à ce mélange 45 g de silice vendue sous la dénomination SILICA BEADS SB 150 par la Société MAPRECOS*. On procède ensuite comme à l'exemple 1. Après lyophilisation, on obtient 45,8 g de poudre brun-gris.

### EXEMPLE 7

On solubilise 7,3 g (0,031 mole) de bromhydrate de 5,6-dihydroxyindoline dans 100 ml de solution aqueuse à 0,1% d'ammoniaque. On ajoute à ce mélange 45 g d'oxyde de titane vendu sous la dénomination F.F. HOMBITAN* par la Société SACHTLEBEN*. On procède ensuite comme à l'exemple 1. Après lyophilisation, on obtient 48,5 g de poudre gris bleuté.

### EXEMPLE 8

On solubilise 4,38 g (0,0188 mole) de bromhydrate de 5,6-dihydroxyindoline et 2,19 g (0,0147 mole) de 5,6-dihydroxyindole dans 100 ml de solution aqueuse à 0,1% d'ammoniaque. On ajoute à ce mélange 45 g de bleu outremer (CI 77007). On agite cette suspension pendant 15 minutes puis on la porte à 80°C. On ajoute alors 6,7 ml de soude 3N et on additionne en 30 minutes 28,6 g d'eau oxygénée contenant 2,3 g (0,067 mole) de peroxyde d'hydrogène en maintenant la température entre 80 et 85°C. L'addition terminée, on maintient la température à 80°C pendant 2 heures puis on refroidit le milieu réactionnel. On centrifuge le produit et on le lave à l'eau. Après lyophilisation, on obtient 49 g de poudre bleu foncé.

### EXEMPLE 9

On solubilise 15,4 g (0,065 mole) de bromhydrate de 5,6-dihydroxyindoline dans 190 ml de solution aqueuse d'ammoniaque à 0,1% et 10 ml d'éthanol. On ajoute à ce mélange 90 g de L-lauroyllysine. On agite cette suspension pendant 15 minutes puis on la porte à 80°C. On ajoute alors 6,6 ml de soude 3N et on additionne en 30 minutes 15,1 g (0,13 moles) de peroxyde d'hydrogène dilué dans 45,3 g d'eau en maintenant la température entre 80°C et 85°C. L'addition terminée, on maintient l'agitation et la température à 80°C pendant environ 2 heures puis on refroidit le milieu réactionnel. On centrifuge le produit et on le lave à l'eau. Après lyophilisation, on obtient 93 g de poudre noire.

### EXEMPLE 10

On solubilise 13,95 g (0,06 mole) de bromhydrate de 5,6-dihydroxyindoline dans 410 ml de solution aqueuse d'ammoniaque à 0,1%. On ajoute à ce mélange 262 g de poly-β-alanine ainsi que 50 ml d'eau. On agite cette suspension pendant 2 heures environ, puis on la porte à 80°C. On ajoute alors 6 ml de soude 3N et on maintient la température à 80°C pendant environ 30 minutes. On ajoute ensuite en 30 minutes, 13,82 g (0,12 mole) de peroxyde d'hydrogène dilué dans 41,46 g d'eau en maintenant la température entre 80°C et 85°C. L'addition terminée, on maintient la température à 80°C et l'agitation pendant environ 2 heures puis on refroidit le milieu réactionnel. On filtre et lave le produit à l'eau. Après lyophilisation, on obtient 93,20 g de poudre brun foncé.

### EXEMPLE 11

On solubilise 7,68 g (0,033 mole) de bromhydrate de 5,6-dihydroxyindoline dans 195 ml de solution aqueuse d'ammoniaque à 0,1%. On ajoute à ce mélange 95 g d'oxychlorure de bismuth vendu sous la dénomination "PEARL GLOW UVR 1086" par la Société MALLINCKRODT*. On ajoute ensuite 3,3 ml de soude 3N. On agite pendant environ 15 minutes puis on porte la température à 80°C.

On additionne alors en 30 minutes 7,55 g (0,07 mole) de peroxyde d'hydrogène dilué dans 22,7 g d'eau en maintenant la température entre 80°C et 85°C. L'addition terminée, on maintient la température à 80°C et l'agitation pendant environ 2 heures, puis on refroidit le milieu réactionnel. On centrifuge le produit et on le lave à l'eau. Après centrifugation, on obtient 95,37 g de poudre brune.

### EXEMPLE 12

On solubilise 7,68 g (0,033 mole) de bromhydrate de 5,6-dihydroxyindoline dans 195 ml de solution aqueuse d'ammoniaque à 0,1%. On ajoute à ce mélange 95 g de poudre constituée d'un mélange Mica/Titane : 83/17. On procède ensuite comme indiqué à l'exemple 11. Après lyophilisation, on obtient 88,21 g de poudre gris métallisé.

### EXEMPLE 13

On solubilise 15,4 g (0,065 mole) de bromhydrate de 5,6-dihydroxyindoline dans 195 ml de solution aqueuse d'ammoniaque à 0,1%. On ajoute ensuite à ce mélange 90 g de microbilles de polymère acrylique vendues sous la dénomination POLYTRAP* par DOW CORNING* ainsi que 10 ml d'éthanol. On procède ensuite comme indiqué à l'exemple 9. Après lyophilisation, on obtient 91,5 g de poudre blanche.

### EXEMPLES DE FORMULATION

### EXEMPLE 1

On prépare un mascara crémeux de formule suivante :

| | |
|---|---|
| - Stéarate de triéthanolamine | 15,0 g |
| - Cire d'abeilles | 8,0 g |
| - Paraffine | 3,0 g |
| - Colophane | 2,0 g |
| - Ozokérite | 10,0 g |
| - Parahydroxybenzoate de propyle | 0,20 g |
| - Parahydroxybenzoate de méthyle | 0,20 g |
| - Gomme arabique | 0,50 g |
| - Hydrolysat de kératine | 1,0 g |
| - Oxyde de fer noir | 5,0 g |
| - Poudre colorée de l'exemple de préparation 4 | 10,0 g |
| - Eau | qsp 100 g |

Le mode opératoire est le suivant :

On fait fondre les cires. On incorpore les pigments . On fait chauffer la phase aqueuse contenant les conservateurs, la gomme et l'hydrolysat de kératine à la même température que la phase cireuse. On mélange les deux phases et on agite vigoureusement. On obtient un mascara crème bleu foncé.

### EXEMPLE 2

On prépare une composition de maquillage des cils suivante :

| | |
|---|---|
| - Copolymère réticulé d'alkylacrylate en C₈-C₃₀/acrylate | 0,10 g |
| - Polymère polycarboxyvinylique réticulé vendu sous la dénomination CARBOPOL* 940 par la Société GOODRICH* | 0,60 g |
| - Triéthanolamine | 0,80 g |
| - Glycérine | 2,0 g |
| - Conservateur | 0,2 g |
| - Octaméthylcyclosiloxane | 25,0 g |
| - Poudre colorée de l'exemple de préparation 3 | 5,0 g |
| - Oxyde de fer noir | 5,0 g |
| - Eau | qsp 100 g |

Les polymères sont dispersés à chaud avec les conservateurs dans l'eau pour faire un gel. La glycérine et la triéthanolamine sont ajoutées. Les pigments sont dispersés dans la silicone et ajoutés dans la phase gel. On obtient une émulsion gélifiée brillante noire pour le maquillage des cils.

### EXEMPLE 3

On prépare un rouge à lèvres de composition suivante :

| | |
|---|---|
| - 2,6-di-tert-butyl-p-crésol | 0,16 g |
| - Lanoline liquide | 17,50 g |
| - Cire microcristalline | 15,0 g |
| - Triglycérides d'acides caprylique et caprique | 11,0 g |
| - Béhénate d'octylglycéryle | 11,0 g |
| - Poudre colorée de l'exemple de préparation 3 | 3,0 g |
| - Mica-titane | 6,0 g |
| - Huile de ricin | qsp 100 g |

On obtient un rouge à lèvres de couleur brun-nacré.

### EXEMPLE 4

On prépare un fard à joues de composition suivante :

| | |
|---|---|
| - Dioxyde de titane | 10,0 g |
| - Mica-titane | 10,0 g |
| - DC Red 30 | 1,2 g |
| - Parahydroxybenzoate de propyle | 0,2 g |
| - Huile de vaseline | 6,0 g |
| - 2-hydroxy 4-méthoxybenzophénone vendu sous la dénomination UVINUL* M40 par la Société BASF* | 0,5 g |
| - Poudre colorée de l'exemple de préparation 6 | 3,0 g |
| - Talc | qsp 100 g |

Ce fard à joues compacté de couleur rose beige s'applique avec un pinceau.

### EXEMPLE 5

On prépare un fard à paupières de composition suivante :

| | |
|---|---|
| - Poudre de polyamide | 15,0 g |
| - Cyclométhicone | 9,0 g |
| - Mica-titane | 30,0 g |
| - Poudre colorée de l'exemple de préparation 5 | 7,0 g |
| - Poudre colorée de l'exemple de préparation 1 | 6,0 g |
| - Talc | qsp 100 g |

Ce fard à paupières de couleur vert-gris s'applique au pinceau ou avec un applicateur en mousse.

### EXEMPLE 6

On prépare une poudre compacte pour le visage de composition suivante :

| | |
|---|---|
| - Poudre de polyéthylène | 5,0 g |
| - Poudre colorée de l'exemple de préparation 3 | 6,0 g |
| - Dioxyde de titane | 10,0 g |
| - Mica | 12,0 g |
| - Myristate d'isopropyle | 1,5 g |
| - Huile de vaseline | 1,5 g |
| - Sorbitol | 0,5 g |
| - Poudre colorée de l'exemple de préparation 7 | 3,0 g |
| - Talc | qsp 100 g |

Cette poudre de couleur beige naturel s'applique avec une houpette ou au pinceau.

### EXEMPLE 7

On prépare un fond de teint pour le visage de composition suivante :

| | |
|---|---|
| - Stéarate de glycéryle | 2,2 g |
| - Mélange d'acides caprique et caprylique et de triester de glycérine | 15,0 g |
| - Oxyde de titane | 10,53 g |
| - Oxyde de fer jaune | 0,83 g |
| - Pigment mélanique synthétique | 0,14 g |
| - Poudre colorée de l'exemple 3 | 0,50 g |
| - Parahydroxybenzoate de méthyle | 0,10 g |
| - Parahydroxybenzoate de propyle | 0,10 g |
| - Conservateur | 0,3 g |
| - 2-hydroxy 4-méthoxybenzophénone | 0,5 g |
| - Octyldiméthyl-p-aminobenzoate | 0,5 g |
| - Silicate de magnésium et d'aluminium | 1,0 g |
| - Triéthanolamine | 1,0 g |
| - Carboxyméthylcellulose | 0,16 g |
| - Sel d'aluminium du produit de réaction de l'anhydride octénylsuccinique et de l'amidon, vendu sous la dénomination DRY FIO* par la Société NATIONAL STARCH* | 5,0 g |
| - Polydiméthylsiloxane cyclique vendu sous la dénomination SILBIONE OIL* 70045 par la Société RHONE POULENC* | 10,0 g |
| - Propylèneglycol | 2,0 g |
| - Glycérine | 3,0 g |
| - Lauroylsarcosinate de sodium | 0,6 g |
| - Acide stéarique | 2,2 g |
| - Eau | qsp 100 g |

Le fond de teint obtenu est beige naturel.

### EXEMPLE 8

On prépare un mascara crémeux de composition suivante :

| | |
|---|---|
| - Stéarate de triéthanolamine | 15,0 g |
| - Cire d'abeilles | 8,0 g |
| - Paraffine | 3,0 g |
| - Colophane | 2,0 g |
| - Ozokérite | 10,0 g |
| - Parahydroxybenzoate de propyle | 0,20 g |
| - Parahydroxybenzoate de méthyle | 0,20 g |
| - Gomme arabique | 0,50 g |
| - Hydrolysat de kératine | 1,0 g |
| - Oxyde de fer noir | 5,0 g |
| - Poudre colorée de l'exemple de préparation 8 | 10,0 g |
| - Eau | qsp 100 g |

Le mode opératoire est le suivant :

On fait fondre les cires. On incorpore les pigments. On fait chauffer la phase aqueuse contenant les conservateurs, la gomme et l'hydrolysat de kératine à la même température que la phase cireuse. On mélange les deux phases et on agite vigoureusement. On obtient un mascara crème bleu foncé.
Les denominations (*) sont à la connaissance de la titulaire et sous toute réserve des marques definées.

## Revendications

1. Produit sous forme de poudre constitué de particules, caractérisé par le fait que les particules sont des particules minérales ou organiques, d'une dimension inférieure à 200 micrometres et comportant dans et/ou sur les particules, un produit indolinique obtenu par polymérisation oxydative mettant en oeuvre au moins une indoline répondant à la formule : 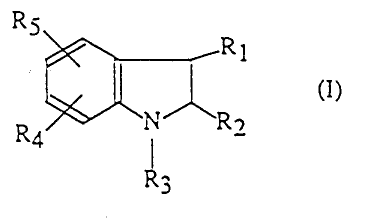 dans laquelle :
R₁ et R₃ représentent, indépendamment l'un de l'autre, un atome d'hydrogène ou un groupement alkyle en C₁-C₄;
R₂ représente un atome d'hydrogène, un groupement alkyle en C₁-C₄, un groupement carboxyle ou alcoxy(C₁-C₄)carbonyle; n radical alkylamino;
R₄ désigne un atome d'hydrogène, un groupement alkyle en C₁-C₄, hydroxyle, alcoxy(C₁-C₄), amino, alkylamino en C₁-C₁₀ ou halogène;
R₅ désigne un atome d'hydrogène, un groupement hydroxyle, alcoxy en C₁-C₄ ou amino;
au moins un des radicaux R₄ ou R₅ désignant un groupement hydroxyle, alcoxy ou amino; sous réserve que lorsque R₅ désigne un groupement amino, R₄ ne peut désigner un radical alkylamino;
R₄ et R₅ peuvent également former un cycle alkylènedioxy en C₁-C₂ et sont en position 5 et 6; ainsi que les sels correspondants.

2. Produit selon la revendication 1, caractérisé par le fait que l'indoline est choisie parmi les composés suivants : la 5,6-dihydroxyindoline, la 6-hydroxyindoline, la 5,6-méthylène-dioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminothdoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline, la 5,6-dihydroxy 2-carboxyindoline.

3. Produit selon les revendications 1 ou 2, caractérisé par le fait que le produit indolinique résulte de la polymérisation oxydative d'au moins une indoline telle que définie dans l'une quelconque des revendications 1 à 3, et d'au moins un indole choisi parmi les monohydroxyindoles, les dihydroxyindoles et les aminoindoles.

4. Produit selon la revendication 3, caractérisé par le fait que les indoles sont choisis parmi les composés répondant à la formule : 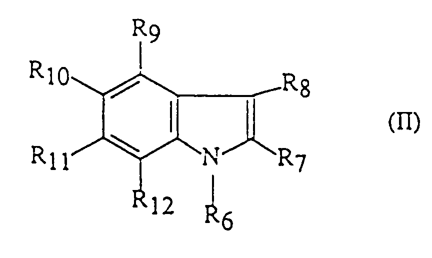 dans laquelle :
R₆ et R₈ désignent, indépendamment l'un de l'autre, un atome d'hydrogène ou un groupe alkyle en C₁-C₄;
R₇ représente un atome d'hydrogène, un groupement alkyle en C₁-C₄, un groupement carboxyle ou un groupement alcoxy(C₁-C₄) carbonyle;
R₉ et R₁₂ désignent, indépendamment l'un de l'autre, un atome d'hydrogène, un groupement hydroxy, un groupe alkyle en C₁-C₄, amino, alcoxy(C₁-C₄), acyl(C₂-C₄)oxy, acyl(C₂-C₄)amino;
R₁₀ désigne hydrogène, un groupement hydroxy, alcoxy(C₁-C₄), alkyle(C₁-C₄), halogène, amino, acyl(C₂-C₁₄)oxy, acyl(C₂-C₄)amino, triméthylsilyloxy;
R₁₁ désigne hydrogène, un groupement hydroxy, alcoxy(C₁-C₄), amino, acyl(C₂-C₄)oxy, acyl(C₂-C₄)amino, triméthylsilyloxy, hydroxyalkyl(C₂-C₄)amino;
R₁₀ et R₁₁ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy;
au moins l'un des groupements R₉ à R₁₂ représente un groupement OZ ou NHR, un seul au plus des groupements R₉ à R₁₂ désignant NHR;
et au plus deux des groupements R₉ à R₁₂ désignent OZ, dans le cas où Z désigne hydrogène, ces groupements sont en position 5 et 6;
et au moins un des groupements R₉ à R₁₂ représente hydrogène, dans le cas où un seul de ces groupements désigne hydrogène, un seul groupement parmi R₉ à R₁₂ désigne alors NHR ou OZ, et les autres groupements désignent alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants.

5. Produit selon l'une quelconque des revendications 1 à 4, caractérisé par le fait que les particules minérales ou organiques sont choisies parmi des particules minérales lamellaires ou non lamellaires, des particules organiques lamellaires ou non lamellaires, les particules minérales ou organiques étant colorées ou non colorées, ces particules ayant une granulométrie moyenne comprise entre 0,01 et 200 micromètres.

6. Produit selon l'une quelconque des revendications 1 à 5, caractérisé par le fait que les particules minérales non lamellaires sont des particules minérales inertes ayant une granulométrie inférieure à micromètres et de préférence inférieure à 10 micromètres

7. Produit selon la revendication 6, caractérisé par le fait que les particules minérales non lamellaires sont des particules de carbonate de calcium, de silice ou d'oxyde de titane.

8. Produit selon l'une quelconque des revendications 1 à 5, caractérisé par le fait que les particules sont des particules lamellaires minérales ou organiques, se présentant sous forme de feuillets éventuellement stratifiés, le rapport entre la plus grande dimension et l'épaisseur étant compris entre 2 et 100, et la dimension la plus grande étant inférieure à 50 micromètres.

9. Produit selon la revendication 8, caractérisé par le fait que les particules lamellaires sont choisies parmi la L-lauroyllysine, les microparticules de céramique éventuellement recouvertes de poudre de zirconium, le dioxyde de titane lamellaire, le talc lamellaire, le nitrure de bore, le mica lamellaire, l'oxychlorure de bismuth, l'oxyde de fer transparent rouge.

10. Produit selon l'une quelconque des revendications 1 à 5, caractérisé par le fait que les particules sont des particules minérales colorées constituées de sels métalliques, insolubles dans le milieu cosmétique, différents des pigments blancs, ayant une granulométrie comprise entre 0,01 et 150 micromètres.

11. Produit selon la revendication 10, caractérisé par le fait que les particules minérales colorées sont choisies parmi les oxydes de fer, le bleu outremer, les oxydes de chrome, le violet de manganèse et le bleu de Prusse.

12. Produit selon l'une quelconque des revendications 1 à 5, caractérisé par le fait que la particule est un pigment nacré ou interférentiel ayant une granulométrie comprise entre 10 et 150 micromètres.

13. Produit selon l'une quelconque des revendications 1 à 5, caractérisé par le fait que les particules organiques sont des particules organiques non lamellaires, ayant une granulométrie inférieure à 100 micrometres et comportant en surface et/ou dans le réseau polymère, le produit indolinique.

14. Produit selon la revendication 13, caractérisé par le fait que les particules de polymères sont choisies parmi les particules de :
a) polymères dérivés de la kératine, éventuellement modifiés;
b) fibroïnes de soie;
c) polymères dérivés de la chitine, éventuellement désacétylés;
d) les polymères cellulosiques;
e) polymères synthétiques choisis parmi :
(i) le polyéthylène, le polypropylène, le polystyrène, le polyméthacrylate de méthyle éventuellement réticulé;
(ii) la poly-β-alanine réticulée;
(iii) les polymères réticulés de styrène/divinylbenzène, de méthacrylate de méthyle/diméthacrylate d'éthylèneglycol ou de stéarate de vinyle/divinylbenzène;
(iv) les microsphères creuses du copolymère de chlorure de vinylidène et d'acrylonitrile;
(v) des microsphères poreuses de polyamide 12, de polyamide 6 ou de copolyamide 6/12;
(vi) de poudre silicone constituée par des gommes, des résines, des élastomères d'organosiloxane.

15. Procédé de préparation d'un produit tel que défini dans l'une quelconque des revendications 1 à 14, caractérisé par le fait que l'on mélange à l'air et à température comprise entre la température ambiante et 100°C, le composé indolinique de formule (I) et éventuellement le composé indolique de formule (II) et les particules minérales ou organiques dans un milieu essentiellement non solvant des particules minérales ou organiques, et qu'on procède ensuite à la formation du produit indolinique par oxydation de l'indoline de formule (I) et éventuellement conjointement de l'indole de formule (II).

16. Procédé selon la revendication 15, caractérisé par le fait que l'oxydation s'effectue lentement à l'air à pH alcalin.

17. Procédé selon la revendication 15, caractérisé par le fait que l'oxydation s'effectue par l'oxygène de l'air en présence d'un catalyseur métallique.

18. Procédé selon la revendication 15, caractérisé par le fait que l'oxydation s'effectue par l'addition d'agents oxydants constitués par le peroxyde d'hydrogène, l'acide periodique et ses sels, le permanganate de potassium, l'hypochlorite de sodium, le persulfate d'ammonium ou l'association iodure/peroxyde d'hydrogène, les peracides organiques ou leurs sels, les chlorites alcalins, le ferricyanure de potassium, l'oxyde d'argent, l'oxyde de plomb, le chlorure ferrique, le nitrite de sodium, les sels de terres rares, les oxydants organiques choisis parmi les ortho et parabenzoquinones, les ortho- et parabenzoquinones monoimines ou diimines, les 1,2- et 1,4-naphtoquinones, les 1,2- et 1,4-naphtoquinones mono- ou diimines.

19. Procédé selon la revendication 18, caractérisé par le fait que l'oxydation s'effectue par le peroxyde d'hydrogène dans un milieu ammoniacal.

20. Procédé selon la revendication 18, caractérisé par le fait que l'oxydation est effectuée par utilisation d'un iodure d'un métal alcalin, alcalino-terreux ou d'ammonium, précédé ou suivi par l'addition de peroxyde d'hydrogène.

21. Procédé selon la revendication 15, caractérisé par le fait que l'on procède à une oxydation par voie enzymatique.

22. Procédé selon l'une quelconque des revendications 15 à 21, caractérisé par le fait que le milieu réactionnel est un milieu essentiellement non solvant des particules et solvant de l'indoline de formule (I) et éventuellement de l'indole de formule (II) et qu'il est constitué par de l'eau ou un mélange d'eau et de solvant.

23. Procédé selon la revendication 22, caractérisé par le fait que le solvant est choisi parmi les alcools inférieurs en C₁-C₄, les alkylèneglycols, les alkyléthers d'alkylèneglycols et le lactate de méthyle.

24. Procédé selon l'une quelconque des revendications 15 à 23, caractérisé par le fait que l'indoline de formule (I) est utilisée dans des proportions comprises entre 0,1 et 10% et de préférence entre 0,5 et 7% en poids, les particules minérales ou organiques représentant 0,075 à 70% et de préférence 4 à 50% en poids, le reste du mélange étant constitué par de l'eau ou un mélange eau/solvant.

25. Utilisation en cosmétique des produits tels que définis dans l'une quelconque des revendications 1 à 14, ou obtenus selon l'une quelconque des revendications 15 à 24.

26. Composition cosmétique, caracterisée par le fait qu'elle contient un produit tel que défini dans l'une quelconque des revendications 1 à 14 ou obtenu selon l'une quelconque des revendications 15 à 24, qu'elle se présente sous forme de lotion, de lotion épaissie, de gel, de crème, de lait, de poudre, de stick, et qu'elle est éventuellement conditionnée en aérosol sous forme de spray ou de mousse.

27. Composition selon la revendication 26, caractérisée par le fait qu'elle est destinée à être utilisée pour le maquillage de la peau, des cheveux, des ongles, des cils et des sourcils et qu'elle se présente sous forme liquide, solide ou pâteuse, anhydre ou aqueuse.

28. Composition selon l'une quelconque des revendications 26 et 27, destinée à la protection de l'épiderme humain contre les rayonnements solaires UV, caractérisée par le fait qu'elle se présente sous forme de suspensions ou de dispersions dans des solvants ou des corps gras, ou sous forme d'émulsions, de pommades, de gels, de bâtonnets solides ou de mousses aérosols.

29. Composition selon l'une quelconque des revendications 26 à 28, caractérisée par le fait qu'elle contient des corps gras, des solvants organiques, des silicones, des épaississants, des adoucissants, des tensio-actifs, des filtres solaires, des agents anti-mousse, des agents hydratants, des parfums, des conservateurs, des agents anti-oxydants, des charges, des séquestrants, des agents de traitement, des propulseurs, des agents alcalinisants ou acidifiants ou d'autres pigments.

30. Composition selon l'une quelconque des revendications 26 à 29, caractérisée par le fait que la poudre sous forme de particules minérales ou organiques comportant dans et/ou sur les particules le produit indolinique, est présente dans des concentrations comprises entre 0,05 et 35% en poids et de préférence entre 0,5 et 20% en poids par rapport au poids total de la composition.

31. Utilisation du produit tel que défini dans l'une quelconque des revendications 1 à 14, ou préparé selon l'une quelconque des revendications 15 à 24, dans la protection de l'épiderme humain.

32. Utilisation du produit tel que défini dans l'une quelconque des revendications 1 à 14, ou préparé selon le procédé défini dans l'une quelconque des revendications 15 à 24, dans des produits de maquillage, de la peau, des cheveux, des ongles, des cils ou des sourcils.

## Claims

1. Product in powder form consisting of particles, characterized in that the particles are inorganic or organic particles less than 200 micrometres in size and which contains, in and/or on the particles, an indoline-based product obtained by oxidative polymerization employing at least one indoline corresponding to the formula: 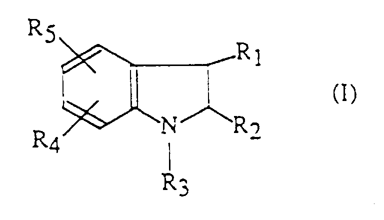 in which:
R₁ and R₃ represent, independently of one another, a hydrogen atom or a C₁-C₄ alkyl group;
R₂ represents a hydrogen atom, a C₁-C₄ alkyl group or a carboxyl or (C₁-C₄ alkoxy)carbonyl group; n alkylamino radical; [sic]
R₄ denotes a hydrogen atom or a C₁-C₄ alkyl, hydroxyl, (C₁-C₄ alkoxy), amino or C₁-C₁₀ alkylamino or halogen group;
R₅ denotes a hydrogen atom or a hydroxyl, C₁-C₄ alkoxy or amino group;
at least one of the radicals R₄ or R₅ denoting a hydroxyl, alkoxy or amino group; with the proviso that when R₅ denotes an amino group, R₄ cannot denote an alkylamino radical;
R₄ and R₅ can also form a C₁-C₂ alkylenedioxy ring, and are at positions 5 and 6;
as well as the corresponding salts.

2. Product according to Claim 1, characterized in that the indoline is chosen from the following compounds: 5,6-dihydroxyindoline 6-hydroxy indoline, 5,6-methylenedioxyindoline, 7-methoxy-6-hydroxyindoline, 6,7-dihydroxyindoline, 5-hydroxy-4-methoxy indoline, 4,5-dihydroxyindoline, 5-methoxy-6-hydroxy indoline, 4-hydroxy-5-methoxyindoline, 5-hydroxy-6-methoxy indoline, 4,7-dihydroxyindoline, 6-aminoindoline, N-ethyl-4-hydroxyindoline, 1-ethyl-6-amino-indoline, 5,6-diaminoindoline, 1-methyl-6-aminoindoline, 2-methyl-6-aminoindoline, 3-methyl-6-aminoindoline, 2-methyl-5,6-diaminoindoline, 5-chloro-7-aminoindoline, 3-methyl-5,7-diaminoindoline, 5,7-diaminoindoline, 2-methyl-5,7-diaminoindoline, 7-aminoindoline, 2-methyl-7-amino indoline, 4-aminoindoline, 4-amino-6-chloroindoline, 4-amino-6-iodoindoline, 4-amino-5-bromoindoline, 4-amino-5-hydroxyindoline, 4-amino-7-hydroxyindoline, 4-amino-5-methoxyindoline, 4-amino-7-methoxyindoline, 5-amino indoline, 2,3-dimethyl-5-aminoindoline, 1-methyl-5-aminoindoline, 2-methyl-5-aminoindoline, 5-[N-(1-methyl hexyl)amino]indoline, 5,6-dimethoxyindoline and 5,6-dihydroxy-2-carboxyindoline.

3. Product according to Claims [sic] 1 or 2, characterized in that the indoline-based product results from the oxidative polymerization of at least one indoline as defined in any one of Claims 1 to 3 [sic] and at least one indole chosen from monohydroxyindoles, dihydroxyindoles and aminoindoles.

4. Product according to Claim 3, characterized in that the indoles are chosen from the compounds corresponding to the formula: 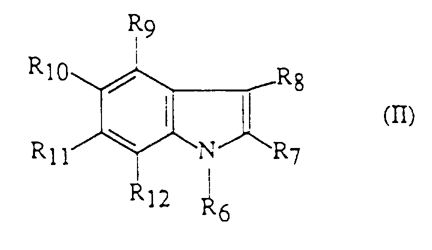 in which:
R₆ and R₈ denote, independently of one another, a hydrogen atom or a C₁-C₄ alkyl group;
R₇ represents a hydrogen atom, a C₁-C₄ alkyl group, a carboxyl group or a (C₁-C₄ alkoxy)carbonyl group;
R₉ and R₁₂ denote, independently of one another, a hydrogen atom, a hydroxyl group or a C₁-C₄ alkyl, amino, (C₁-C₄ alkoxy), (C₂-C₄ acyl)oxy or (C₂-C₄ acyl)amino group;
R₁₀ denotes hydrogen or a hydroxyl, (C₁-C₄ alkoxy), (C₁-C₄ alkyl), halogen, amino, (C₂-C₁₄ acyl)oxy, (C₂-C₄ acyl)-amino or trimethylsilyloxy group;
R₁₁ denotes hydrogen or a hydroxyl, (C₁-C₄ alkoxy), amino, (C₂-C₄ acyl)oxy, (C₂-C₄ acyl)amino, trimethylsilyloxy or hydroxy(C₂-C₄ alkyl)amino group;
R₁₀ and R₁₁, together with the carbon atoms to which they are attached, can form a methylenedioxy ring optionally substituted with a C₁-C₄ alkyl or C₁-C₄ alkoxy group or a carbonyldioxy ring;
at least one of the groups R₉ to R₁₂ represents a group OZ or NHR, not more than one of the groups R₉ to R₁₂ denoting NHR;
and not more than two of the groups R₉ to R₁₂ denote OZ, in the case where Z denotes hydrogen, these groups are at positions 5 and 6;
and at least one of the groups R₉ to R₁₂ represents hydrogen, in the case where only one of these groups denotes hydrogen, only one group from among R₉ to R₁₂ then denotes NHR or OZ and the other groups denote C₁-C₄ alkyl;
R in NHR denoting a hydrogen atom or a C₂-C₄ acyl or C₂-C₄ hydroxyalkyl group, and Z in OZ denoting a hydrogen atom or a C₂-C₁₄ acyl, C₁-C₄ alkyl or trimethylsilyl group;
and the corresponding salts.

5. Product according to any one of Claims 1 to 4, characterized in that the inorganic or organic particles are chosen from lamellar or non-lamellar inorganic particles and lamellar or non-lamellar organic particles, the inorganic or organic particles being coloured or uncoloured, these particles having an average particle size of between 0.01 and 200 micrometres.

6. Product according to any one of Claims 1 to 5, characterized in that the non-lamellar inorganic particles are inert inorganic particles having a particle size of less than 20 micrometres, and preferably less than 10 microns.

7. Product according to Claim 6, characterized in that the non-lamellar inorganic particles are calcium carbonate, silica or titanium oxide particles.

8. Product according to any one of Claims 1 to 5, characterized in that the particles are inorganic or organic lamellar particles which take the form of lamellae, where appropriate stratified, the ratio of the largest dimension to the thickness being between 2 and 100 and the largest dimension being less than 50 micrometres.

9. Product according to Claim 8, characterized in that the lamellar particles are chosen from L-lauroyllysine, microparticles of ceramic which are optionally coated with zirconium powder, lamellar titanium dioxide, lamellar talc, boron nitride, lamellar mica, bismuth oxychloride and transparent red iron oxide.

10. Product according to any one of Claims 1 to 5, characterized in that the particles are coloured inorganic particles consisting of metal salts which are insoluble in the cosmetic medium, other than white pigments, having a particle size of between 0.01 and 150 micrometres.

11. Product according to Claim 10, characterized in that the coloured inorganic particles are chosen from iron oxides, ultramarine blue, chromium oxides, manganese violet and Prussian blue.

12. Product according to any one of Claims 1 to 5, characterized in that the particle is a nacreous or interference pigment having a particle size of between 10 and 150 micrometres.

13. Product according to any one of Claims 1 to 5, characterized in that the organic particles are non-lamellar organic particles having a particle size of less than 100 micrometres and which contain the indoline-based product at the surface and/or in the polymer network.

14. Product according to Claim 13, characterized in that the particles of polymers are chosen from particles of:
a) polymers derived from keratin, optionally modified;
b) silk fibroins;
c) polymers derived from chitin, optionally deacetylated;
d) cellulose polymers;
e) synthetic polymers chosen from:
(i) polyethylene, polypropylene, polystyrene and poly(methyl methacrylate) which is optionally crosslinked;
(ii) crosslinked poly-β-alanine;
(iii) styrene/divinylbenzene, methyl methacrylate/ethylene glycol dimethacrylate or vinyl stearate/divinylbenzene crosslinked polymers;
(iv) hollow microspheres of the copolymer of vinylidene chloride and acrylonitrile;
(v) porous microspheres of polyamide 12, polyamide 6 or copolyamide 6/12;
(vi) silicone powder consisting of gums, resins and organosiloxane elastomers.

15. Process for preparing a product as defined in any one of Claims 1 to 14, characterized in that the indoline compound of formula (I) and, where appropriate, the indole compound of formula (II) and the inorganic or organic particles are mixed in the air and at a temperature between room temperature and 100°C in a medium which is essentially a non-solvent for the inorganic or organic particles, and in that the formation of the indoline-based product is then carried out by oxidation of the indolene-based product is then carried out by oxidation of the indoline of formula (I) and, where appropriate, jointly, of the indole of formula (II).

16. Process according to Claim 15, characterized in that the oxidation is performed slowly in the air at an alkaline pH.

17. Process according to Claim 15, characterized in that the oxidation is performed with aerial oxygen in the presence of a metal-based catalyst.

18. Process according to Claim 15, characterized in that the oxidation is performed by adding oxidizing agents consisting of hydrogen peroxide, periodic acid and its salts, potassium permanganate, sodium hypochlorite, ammonium persulphate or the iodide/hydrogen peroxide combination, organic peracids or their salts, alkali metal chlorites, potassium ferricyanide, silver oxide, lead oxide, ferric chloride, sodium nitrite, rare-earth salts or organic oxidizing agents chosen from ortho- and para-benzoquinones, ortho- and para-benzoquinone mono-imines or diimines, 1,2- and 1,4-naphthoquinones and 1,2- and 1,4-naphthoquinone mono- or diimines.

19. Process according to Claim 18, characterized in that the oxidation is performed with hydrogen peroxide in an ammoniacal medium.

20. Process according to Claim 18, characterized in that the oxidation is performed by the use of an alkali metal iodide, alkaline-earth metal iodide or ammonium iodide, preceded or followed by the addition of hydrogen peroxide.

21. Process according to Claim 15, characterized in that an enzymatic oxidation is carried out.

22. Process according to any one of Claims 15 to 21, characterized in that the reaction medium is a medium which is essentially a non-solvent for the particles and solvent for the indoline of formula (I) and, where appropriate, for the indole of formula (II), and in that it consists of water or a mixture of water and solvent.

23. Process according to Claim 22, characterized in that the solvent is chosen from C₁-C₄ lower alcohols, alkylene glycols, alkylene glycol alkyl ethers and methyl lactate.

24. Process according to any one of Claims 15 to 23, characterized in that the indoline of formula (I) is used in proportions of between 0.1 and 10 %, and preferably between 0.5 and 7 %, by weight, the inorganic or organic particles representing 0.075 to 70 %, and preferably 4 to 50 %, by weight, the remainder of the mixture consisting of water or a water/solvent mixture.

25. Use in cosmetics of the products as defined in any one of Claims 1 to 14, or obtained according to any one of Claims 15 to 24.

26. Cosmetic composition, characterized in that it contains a product as defined in any one of Claims 1 to 14 or obtained according to any one of Claims 15 to 24, in that it takes the form of a lotion, thickened lotion, gel, cream, milk, powder or stick, and in that it is optionally packaged as an aerosol in the form of a spray or foam.

27. Composition according to Claim 26, characterized in that it is intended for use for making up the skin, hair, nails, eyelashes and eyebrows, and in that it takes anhydrous or aqueous pasty, solid or livid form.

28. Composition according to either of Claims 26 and 27, intended for protecting the human epidermis against solar UV radiation, characterized in that it takes the form of suspensions or dispersions in solvents or fats, or the form of emulsions, ointments, gels, solid sticks or aerosol foams.

29. Composition according to any one of Claims 26 to 28, characterized in that it contains fats, organic solvents, silicones, thickeners, demulcents, surfactants, sunscreen agents, antifoams, hydrating agents, perfumes, preservatives, antioxidants, fillers, sequestering agents, treatment agents, propellants, alkalinizing or acidifying agents or other pigments.

30. Composition according to any one of Claims 26 to 29, characterized in that the powder in the form or inorganic or organic particles which contain the indoline-based product in and/or on the particles is present in concentrations of between 0.05 and 35 % by weight, and preferably between 0.5 and 20 % by weight, relative to the total weight of the composition.

31. Use of the product as defined in any one of Claims 1 to 14, or prepared according to any one of Claims 15 to 24, in protecting the human epidermis.

32. Use of the product as defined in any one of Claims 1 to 14, or prepared according to the process defined in any one of Claims 15 to 24, in makeup products for the skin, hair, nails, eyelashes or eyebrows.

## Patentansprüche

1. Produkt in Form eines Pulvers aus Partikeln,
dadurch **gekennzeichnet**, daß
die Partikel mineralische oder organische Partikel einer Größe von weniger als 200 µm sind und in und/oder auf den Partikeln ein Indolin-Produkt aufweisen, das durch oxidative Polymerisation erhältlich ist, wobei man mindestens ein Indolin der Formel: 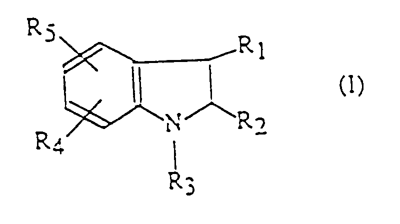 worin gilt:
R₁ und R₃ stellen, unabhängig voneinander, ein Wasserstoffatom oder eine C₁₋₄-Alkylgruppe dar;
R₂ stellt ein Wasserstoffatom, eine C₁₋₄-Alkyl-, Carboxyl- oder eine C₁₋₄-Alkoxycarbonylgruppe dar;
R₄ bedeutet ein Wasserstoffatom, eine C₁₋₄-Alkyl-, Hydroxyl-, C₁₋₄-Alkoxy-, Amino-, C₁₋₁₀-Alkylaminogruppe oder ein Halogenatom;
R₅ bedeutet ein Wasserstoffatom, eine Hydroxyl-, C₁₋₄-Alkoxy- oder eine Aminogruppe,
wobei mindestens einer der Reste R₄ oder R₅ eine Hydroxyl-, Alkoxy- oder Aminogruppe bedeutet, mit der Maßgabe, daß, wenn R₅ eine Aminogruppe bedeutet, R₄ keinen Alkylaminorest bedeutet;
R₄ und R₅ können auch einen C₁₋₂-Alkylendioxy-Ring bilden und befinden sich dann in Position 5 und 6,
sowie die entsprechenden Salze
einsetzt.

2. Produkt gemäß Anspruch 1,
dadurch **gekennzeichnet**, daß
das Indolin aus den folgenden Verbindungen ausgewählt ist: 5,6-Dihydroxyindolin, 6-Hydroxyindolin, 5,6-Methylendioxyindolin, 7-Methoxy-6-hydroxyindolin, 6,7-Dihydroxyindolin, 5-Hydroxy-4-methoxyindolin, 4,5-Dihydroxyindolin, 5-Methoxy-6-hydroxyindolin, 4-Hydroxy-5-methoxyindolin, 5-Hydroxy-6-methoxyindolin, 4,7-Dihydroxyindolin, 6-Aminoindolin, N-Ethyl-4-hydroxyindolin, 1-Ethyl-6-aminoindolin, 5,6-Diaminoindolin, 1-Methyl-6-aminoindolin, 2-Methyl-6-aminoindolin, 3-Methyl-6-aminoindolin, 2-Methyl-5,6-diaminoindolin, 5-Chlor-7-aminoindolin, 3-Methyl-5,7-diaminoindolin, 5,7-Diaminoindolin, 2-Methyl-5,7-diaminoindolin, 7-Aminoindolin, 2-Methyl-7-aminoindolin, 4-Aminoindolin, 4-Amino-6-chlorindolin, 4-Amino-6-jodindolin, 4-Amino-5-bromindolin, 4-Amino-5-hydroxyindolin, 4-Amino-7-hydroxyindolin, 4-Amino-5-methoxyindolin, 4-Amino-7-methoxyindolin, 5-Aminoindolin, 2,3-Dimethyl-5-aminoindolin, 1-Methyl-5-aminoindolin, 2-Methyl-5-aminoindolin, 5-N-(1-Methylhexyl)aminoindolin, 5,6-Dimethoxyindolin und aus 5,6-Dihydroxy-2-carboxyindolin.

3. Produkt gemäß den Ansprüchen 1 oder 2,
dadurch **gekennzeichnet**, daß
das Indolin-Produkt aus der oxidativen Polymerisation mindestens eines in jedem der Ansprüche 1 oder 2 definierten Indolins und mindestens eines Indols entsteht, das aus Monohydroxyindolen, Dihydroxyindolen und aus Aminoindolen ausgewählt ist.

4. Produkt gemäß Anspruch 3,
dadurch **gekennzeichnet**, daß
die Indole aus Verbindungen der Formel: 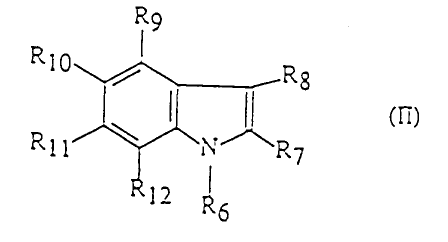 worin gilt:
R₆ und R₈ bedeuten, unabhängig voneinander, ein Wasserstoffatom oder eine C₁₋₄-Alkylgruppe;
R₇ stellt ein Wasserstoffatom, eine C₁₋₄-Alkyl-, Carboxyl- oder eine C₁₋₄-Alkoxycarbonylgruppe dar;
R₉ und R₁₂ bedeuten, unabhängig voneinander, ein Wasserstoffatom, eine Hydroxy-, C₁₋₄-Alkyl-, Amino-, C₁₋₄-Alkoxy-, C₂₋₄-Acyloxy- oder eine C₂₋₄-Acylaminogruppe;
R₁₀ bedeutet Wasserstoff, eine Hydroxy-, C₁₋₄-Alkoxy-, C₁₋₄-Alkyl-, Halogen-, Amino-, C₂₋₁₄-Acylloxy-, C₂₋₄-Acylamino- oder eine Trimethylsilyloxygruppe;
R₁₁ bedeutet Wasserstoff, eine Hydroxy-, C₁₋₄-Alkoxy-, Amino-, C₂₋₄-Acyloxy-, C₂₋₄-Acylamino-, Trimethylsilyloxy- oder eine C₂₋₄-Hydroxyalkylaminogruppe;
R₁₀ und R₁₁ können, zusammen mit den Kohlenstoffatomen, an die sie gebunden sind, auch einen Methylendioxy-Ring bilden, der gegebenenfalls mit einer C₁₋₄-Alkyl- oder einer C₁₋₄-Alkoxygruppe oder einem Carbonyldioxy-Ring substituiert ist;
mindestens eine der Gruppen R₉ bis R₁₂ stellt eine OZ- oder NHR-Gruppe dar, wobei höchstens eine der Gruppen R₉ bis R₁₂ NHR bedeutet;
höchstens zwei der Gruppen R₉ bis R₁₂ bedeuten OZ, wobei wenn Z Wasserstoff bedeutet, diese Gruppen in Position 5 und 6 vorliegen;
mindestens eine der Gruppen R₉ bis R₁₂ stellt Wasserstoff dar, wobei, wenn nur eine dieser Gruppen Wasserstoff bedeutet, eine Gruppe aus R₉ bis R₁₂ dann NHR oder OZ und die anderen Gruppen einen C₁₋₄-Alkylrest bedeuten,
wobei R in NHR ein Wasserstoffatom, eine C₂₋₄-Acyl- oder eine C₂₋₄-Hydroxyalkylgruppe und Z in OZ ein Wasserstoffatom, eine C₂₋₁₄-Acyl-, C₁₋₄-Alkyl- oder eine Trimethylsilylgruppe bedeuten,
und aus den entsprechenden Salzen ausgewählt sind.

5. Produkt gemäß jedem der Ansprüche 1 bis 4,
dadurch **gekennzeichnet**, daß
die mineralischen oder organischen Partikel aus lamellaren oder nicht-lamellaren mineralischen Partikeln, lamellaren oder nicht-lamellaren organischen Partikeln und aus mineralischen oder organischen Partikeln, die gefärbt sind oder nicht, ausgewählt sind, wobei diese Partikel eine mittlere Korngröße von 0,01 bis 200 µm aufweisen.

6. Produkt gemäß jedem der Ansprüche 1 bis 5,
dadurch **gekennzeichnet**, daß
die mineralischen nicht-lamellaren Partikel inerte mineralische Partikel mit einer Korngröße von weniger als 20 und vorzugsweise von weniger als 10 µm sind.

7. Proukt gemäß Anspruch 6,
dadurch **gekennzeichnet**, daß
die nicht-lamellaren mineralischen Partikel Partikel aus Calciumcarbonat, Kieselsäure oder Titanoxid sind.

8. Produkt gemäß jedem der Ansprüche 1 bis 5,
dadurch **gekennzeichnet**, daß
die Partikel mineralische oder organische lamellare Partikel sind, die in Form gegebenenfalls abgeflachter Blättchen vorliegen, wobei das Verhältnis der größten Länge zur Dicke 2 bis 100 und die größte Länge weniger als 50 µm betragen.

9. Produkt gemäß Anspruch 8,
dadurch **gekennzeichnet**, daß
die lamellaren Partikel aus L-Lauryollysin, aus gegebenenfalls mit Zirkoniumpulver überzogenen Mikropartikeln aus Keramik, aus lamellarem Titandioxid, lamellarem Talkum, Bornitrid, lamellarem Glimmer, Wismutoxichlorid und aus rotem durchsichtigen Eisenoxid ausgewählt sind.

10. Proudkt gemäß jedem der Ansprüche 1 bis 5,
dadurch **gekennzeichnet**, daß
die Partikel gefärbte mineralische Partikel aus Metallsalzen sind, die im kosmetischen Milieu unlöslich sind und sich von weißen Pigmetnen unterscheiden, wobei die Partikel eine Korngröße von 0,01 bis 150 µm aufweisen.

11. Produkt gemäß Anspruch 10,
dadurch **gekennzeichnet**, daß
die gefärbten mineralischen Partikel aus Eisenoxiden, Ultramarinblau, Chromoxiden, Mangan-Violett und aus Preussisch-Blau ausgewählt sind.

12. Produkt gemäß jedem der Ansprüche 1 bis 6,
dadurch **gekennzeichnet**, daß
die Partikel Perlmutt- oder Interferenz-Partikel einer Korngröße von 10 bis 150 µm sind.

13. Produkt gemäß jedem der Ansprüche 1 bis 5,
dadurch **gekennzeichnet**, daß
die organischen Partikel nicht-lamellare organische Partikel einer Korngröße von weniger als 100 µm sind und auf der Oberfläche und/oder im Polymerharz das Indolin-Produkt aufweisen.

14. Produkt gemäß Anspruch 13,
dadurch **gekennzeichnet**, daß
die Partikel aus Polymeren ausgewählt sind aus Partikeln aus:
a) von Keratin abgeleiteten Polymeren, die gegebenenfalls modifiziert sind;
b) Seiden-Fibroinen;
c) von Chitin abgeleiteten Polymeren, die gegebenenfalls entacetyliert sind;
d) Cellulose-Polymeren;
e) synthetischen Polymeren, die ausgewählt sind aus:
(i) Polyethylen, Polypropylen, Polystyrol, gegebenenfalls vernetztem Polymethylmethacrylat;
(ii) vernetztem Poly-β-alanin;
(iii) vernetzten Polymeren aus Styrol/Divinylbenzol, Methalmethacrylat/Ethylenglycoldimethacrylat oder aus Vinylstearat/Divinylbenzol;
(iv) Mikrohohlkugeln des Copolymers aus Vinylidenchlorid und Acrylnitril;
(v) porösen Mikrokugeln aus Polyamid 12, Polyamid 6 oder Copolyamid 6/12;
(vi) Silikon-Pulver aus Gummi-, Harzprodukten und Elastomeren aus Organosiloxan.

15. Verfahren zur Herstellung eines in jedem der Ansprüche 1 bis 14 definierten Produkts,
dadurch **gekennzeichnet**, daß
man an der Luft und bei einer Temperatur von Umgebungstemperatur bis 100°C die Indolin-Verbindung der Formel (I) und gegebenenfalls die Indolverbindung der Formel (II) und die mineralischen oder organischen Partikel in einem Milieu aus einem im wesentlichen Nicht-Lösungsmittel für die mineralischen oder organischen Partikel vermischt und man dann das Indolin-Produkt durch Oxidation des Indolins der Formel (I) gegebenenfalls zusammen mit dem Indol der Formel (II) bildet.

16. Verfahren gmeäß Anspruch 15,
dadurch **gekennzeichnet**, daß
die Oxidation langsam an der Luft bei alkalischem pH-Wert durchgeführt wird.

17. Verfahren gemäß Anspruch 15,
dadurch **gekennzeichnet**, daß
die Oxidation mit Luftsauerstoff in Gegenwart eines metallhaltigen Katalysators durchgeführt wird.

18. Verfahren gemäß Anspruch 15,
dadurch **gekennzeichnet**, daß
die Oxidation durch Zugabe oxidierender Mittel aus Wasserstoffperoxid, Perjodsäure und ihren Salzen, Kaliumpermanganat, Natriumhypochlorit, Anmoniumpersulfat oder der Mischung aus Jodid/Wasserstoffperoxid, organischen Persäuren oder deren Salzen, Alkalichloriten, Kaliumferricyand, Silberoxid, Bleioxid, Eisen(III)chlorid, Natriumnitrit, Salzen seltener Erden, organischen oxidierenden Mitteln aus o- und p-Benzochinonen, o- und p-Benzochinonmonoiminen oder -diiminen, 1,2- und 1,4-Naphthochinonen und aus 1,2- und 1,4-Naphthochinonmono- oder -diiminen durchgeführt wird.

19. Verfahren gemäß Anspruch 18,
dadurch **gekennzeichnet**, daß
die Oxidation mit Wasserstoffperoxid in ammoniakalischem Milieu durchgeführt wird.

20. Verfahren gemäß Anspruch 18,
dadurch **gekennzeichnet**, daß
die Oxidation durch Verwendung von Alkali-, Erdalkali- oder Ammoniumjodid und anschließender Zugabe von Wasserstoffperoxid durchgeführt wird.

21. Verfahren gemäß Anspruch 15,
dadurch **gekennzeichnet**, daß
man eine Oxidation auf enzymatischem Weg durchführt.

22. Verfahren gemäß jedem der Ansprüche 15 bis 21,
dadurch **gekennzeichnet**, daß
das Reaktionsmilieu ein Milieu aus einem im wesentlichen Nicht-Lösungsmittel für die Partikel und einem Lösungsmittel für das Indolin der Formel (I) und gegebenenfalls das Indol der Formel (II) ist und aus Wasser oder einer Mischung aus Wasser und Lösungsmittel zusammengesetzt ist.

23. Verfahren gemäß Anspruch 22,
dadurch **gekennzeichnet**, daß
das Lösungsmittel aus C₁₋₄-Niedrigalkoholen, Alkylenglycolen, Alkylethern von Alkylenglycol und aus Methyllactat ausgewählt ist.

24. Verfahren gemäß jedem der Ansprüche 15 bis 23,
dadurch **gekennzeichnet**, daß
das Indolin der Formel (I) in Mengenanteilen von 0,1 bis 10 und vorzugsweise von 0,5 bis 7 Gew.% eingesetzt wird, wobei die mineralischen oder organischen Partikel 0,075 bis 70 und vorzugsweise 4 bis 50 Gew.% ausmachen, wobei der Rest der Mischung aus Wasser oder einer Mischung aus Wasser/Lösungsmittel zusammengesetzt ist.

25. In der Kosmetik vorgesehene Verwendung der in jedem der Ansprüche 1 bis 14 defnierten oder gemäß jedem der Ansprüche 15 bis 24 erhältlichen Produkte.

26. Kosmetische Zusammensetzung
dadurch **gekennzeichnet**, daß
sie ein in jedem der Ansprüche 1 bis 14 definiertes oder gemäß jedem der Ansprüche 15 bis 24 erhältliches Produkt enthält und in Form einer Lotion, verdickten Lotion, eines Gels, einer Creme, Milch, eines Puders oder Pulvers, Stifts oder Stabs vorliegt und gegebenenfalls als Aerosol in Form eines Spray oder Schaum zubereitet ist.

27. Zusammensetzung gemäß Anspruch 26,
dadurch **gekennzeichnet**, daß
sie dazu bestimmt ist, zum Schmnken der Haut, der Haare, der Nägel, der Wimpern und Augenbrauen verwendet zu werden, und in flüssiger, fester oder pastenartiger, wasserfreier oder wässriger Form vorliegt.

28. Zusammensetzung gemäß einem der Ansprüche 26 und 27 zum Schutz der menschlichen Epidermis vor UV-Sonnenstrahlungen,
dadurch **gekennzeichnet**, daß
sie in Form von Suspensionen oder Dispersionen in Lösungsmitteln oder Fettkörpern oder in Form von Emulsionen, Pomaden, Gelen, Feststoffstäbchen oder Aerosol-Schäumen vorliegt.

29. Zusammensetzung gemäß einem der Ansprüche 26 bis 28,
dadurch **gekennzeichnet**, daß
sie Fettkörper, organische Lösungsmittel, Silikone, Verdickungsmittel, Weichmacher, oberflächenaktive Mittel, Sonnenfilterstoffverbindungen, Antischaummittel, Hydratisiermittel, Parfüm-Produkte, Konservierungsmittel, Antioxidantien, Beaufschlagungsmittel, Sequestriermittel, Behandlungsmittel, Treibmittel, alkalisch oder sauer stellende Mittel oder weitere Pigmente enthält.

30. Zusammensetzung gemäß einem der Ansprüche 26 bis 29,
dadurch **gekennzeichnet**, daß
das Pulver in Form der mineralischen oder organischen Partikel, welche in und/oder auf den Partikeln das Indolin-Produkt aufweisen, in Konzentrationen von 0,05 bis 35 und vorzugsweise von 0,5 bis 20 Gew.% vorhanden ist, bezogen auf das Gesamtgewicht der Zusammensetzung.

31. Verwendung des in jedem der Ansprüche 1 bis 14 definierten oder gemäß jedem der Ansprüche 15 bis 24 herstellbaren Produkts zum Schutz der menschlichen Epidermis.

32. Verwendung des in jedem der Ansprüche 1 bis 14 definierten oder gemäß jedem der Ansprüche 15 bis 24 herstellbaren Produkts in Produkten zum Schminken der Haut, der Haare, Nägel, Wimpern oder Augenbrauen.
